(19) Europäisches Patentamt / European Patent Office / Office européen des brevets

(11) **EP 4 513 156 A1**

(12) **EUROPEAN PATENT APPLICATION**
published in accordance with Art. 153(4) EPC

(43) Date of publication:
**26.02.2025 Bulletin 2025/09**

(21) Application number: **22938106.6**

(22) Date of filing: **24.06.2022**

(51) International Patent Classification (IPC):
*G01L 1/14* (2006.01)   *G01B 7/00* (2006.01)
*A47C 17/86* (2006.01)   *A47C 23/00* (2006.01)
*A47C 27/14* (2006.01)

(86) International application number:
**PCT/CN2022/101135**

(87) International publication number:
**WO 2023/201877 (26.10.2023 Gazette 2023/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.04.2022   CN 202210418377**

(71) Applicant: De Rucci Healthy Sleep Co., Ltd.
Dongguan, Guangdong 523000 (CN)

(72) Inventor: WANG, Bingkun
Dongguan, Guangdong 523000 (CN)

(74) Representative: TBK
Bavariaring 4-6
80336 München (DE)

(54) **FLEXIBLE SENSOR AND INTELLIGENT MAT**

(57)     Provided are a flexible sensor (10) and an intelligent mat (20). The flexible sensor (10) is electrically connected to an external processing circuit. The flexible sensor (10) includes a first electrode plate (100), a second electrode plate (200), and an elastic medium (300). The first electrode plate (100) includes multiple first conductive strips (110) spaced apart, and adjacent first conductive strips (110) are electrically connected to each other. The first electrode plate (100) and the second electrode plate (200) are disposed on two opposite sides of the elastic medium (300). The second electrode plate (200) and the multiple first conductive strips (110) form a variable capacitor with the elastic medium (300) as an insulating layer. The elastic medium (300) undergoes elastic deformation when being subjected to pressure to change the distance between the first electrode plate (100) and the second electrode plate (200).

**FIG. 1**

**Description**

This application claims priority to Chinese Patent Application No. 202210418377.3 filed with the China National Intellectual Property Administration (CNIPA) on Apr. 20, 2022, the disclosure of which is incorporated herein by reference in its entirety.

TECHNICAL FIELD

The present application relates to the technical field of flexible sensors, for example, to a flexible sensor and an intelligent mat.

BACKGROUND

With the development of technology, many pieces of intelligent furniture to which technology is applied have appeared on the market, such as intelligent massage chairs, intelligent mattresses, intelligent couches, and car seat cushions. These pieces of intelligent furniture also have a function of detecting whether users are using them. This detection function is generally completed using sensors.

Sensors used include thin-film piezoresistive pressure sensors, vibration sensors, fiber-optic pressure sensors, pressure switch sensors, and air pressure sensors. However, these sensors have certain limitations such as high cost, complex algorithms, poor comfort, susceptibility to environmental interference, poor reliability, and strong foreign body sensation and thereby have not been widely used.

SUMMARY

The present application provides a flexible sensor and an intelligent mat to solve the limitations of pressure sensors in intelligent furniture.

The present application provides a flexible sensor. The flexible sensor is configured to be electrically connected to an external processing circuit and includes a first electrode plate, a second electrode plate, and an elastic medium.

The first electrode plate includes a plurality of first conductive strips spaced apart; adjacent first conductive strips of the plurality of first conductive strips are electrically connected to each other.

The first electrode plate and the second electrode plate are disposed on two opposite sides of the elastic medium; the second electrode plate and the plurality of first conductive strips form a variable capacitor with the elastic medium as an insulating layer.

In a case where the elastic medium is subjected to pressure, the elastic medium is configured to undergo elastic deformation to change a distance between the first electrode plate and the second electrode plate.

The present application provides an intelligent mat including at least one flexible sensor described in the preceding.

BRIEF DESCRIPTION OF DRAWINGS

FIG. 1 is a diagram illustrating the structure of a flexible sensor according to an embodiment of the present application.

FIG. 2 is a diagram illustrating the bottom structure of a flexible sensor according to an embodiment of the present application.

FIG. 3 is another diagram illustrating the bottom structure of a flexible sensor according to an embodiment of the present application.

FIG. 4 is a diagram illustrating the cross-sectional structure of a flexible sensor according to an embodiment of the present application.

FIG. 5 is a diagram illustrating the top structure of a flexible sensor according to an embodiment of the present application.

FIG. 6 is another diagram illustrating the top structure of a flexible sensor according to an embodiment of the present application.

FIG. 7 is another diagram illustrating the top structure of a flexible sensor according to an embodiment of the present application.

FIG. 8 is another diagram illustrating the top structure of a flexible sensor according to an embodiment of the present application.

FIG. 9 is another diagram illustrating the top structure of a flexible sensor according to an embodiment of the present application.

FIG. 10 is a diagram illustrating the structure of an intelligent mat according to an embodiment of the present application.

DETAILED DESCRIPTION

Technical solutions in embodiments of the present application are described in conjunction with drawings in the embodiments of the present application. The embodiments described herein are part, not all, of the embodiments of the present application.

In the present application, terms such as "first" and "second" are used for distinguishing between similar objects and are not necessarily used for describing a particular order or sequence. Data used in this manner are interchangeable where appropriate so that the embodiments of the present application described herein can be implemented in an order not illustrated or described herein. Additionally, terms "including", "having" and any other variations thereof are intended to encompass a non-exclusive inclusion. For example, a process, method, system, product, or device that includes a series of steps or units not only includes the expressly listed steps or units but may also include other steps or units that are not expressly listed or are inherent to such process, method, product, or device.

Pressure sensors have their own limitations. For example, a vibration sensor has a limited installation location, complex structural design and processing techniques, a high cost, and a slow detection speed. Whether someone is using intelligent furniture provided with the vibration sensor is determined only by sensing heart rate and breathing through the sensor. This often requires several cycles of heart rate or breathing data, and then the heart rate or breathing is measured through an algorithm to determine whether someone is using the intelligent furniture. The vibration sensor has a slow response speed and a complex algorithm and is susceptible to external environmental interference. If the intelligent furniture is a double bed, two people interfere with each other, resulting in worse accuracy of the vibration sensor. If a pressure switch sensor is used for detection, this belongs to point detection and requires multiple pressure sensors to be installed in the intelligent furniture, which involves a large number of sensors, has complex wires and a high cost and is easy to crush the sensor and difficult to manufacture. If a thin-film piezoresistive pressure sensor is used, the cost is relatively high, and films are easy to break when an excessively large force is applied. If a fiber-optic sensor is used for being woven into the intelligent furniture, a special optical fiber must be used, so the detection cost is high; the optical fiber cannot have an excessively large bending angle after being applied with a force; the detection and application scope is limited; the subsequent maintenance cost is also relatively high. If an air pressure sensor is used, multiple air bags must be used, so the detection cost is high, a circuit is complex, and the air pressure sensor detection is susceptible to the environment air pressure and the environment temperature.

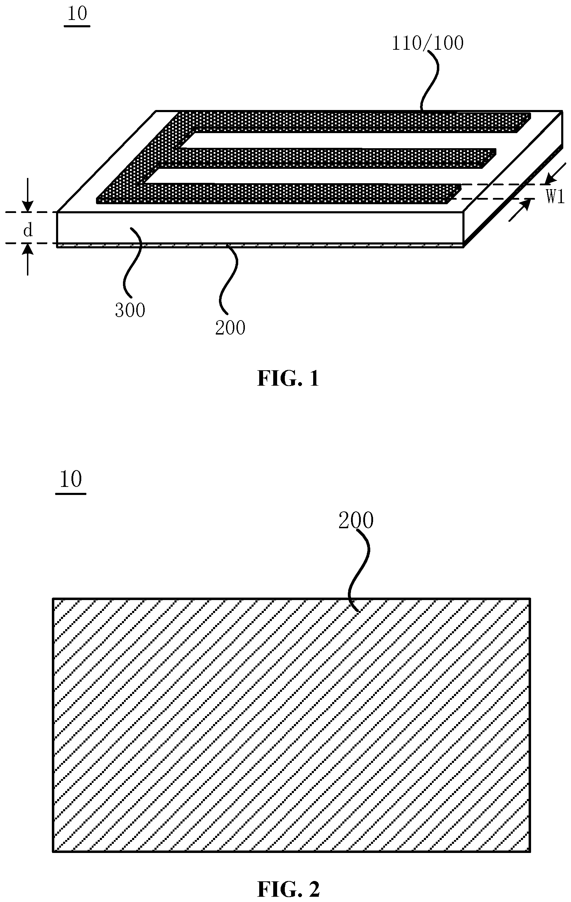

An embodiment of the present application provides a flexible sensor. FIG. 1 is a diagram illustrating the structure of a flexible sensor according to an embodiment of the present application. As shown in FIG. 1, the flexible sensor 10 includes a first electrode plate 100, a second electrode plate 200, and an elastic medium 300; the first electrode plate 100 includes multiple first conductive strips 110 spaced apart, and adjacent first conductive strips 110 are electrically connected to each other; the first electrode plate 100 and the second electrode plate 200 are disposed on two opposite sides of the elastic medium 300; the second electrode plate 200 and the multiple first conductive strips 110 form a variable capacitor with the elastic medium 300 as an insulating layer.

The elastic medium 300 undergoes elastic deformation when being subjected to pressure to change the distance between the first electrode plate 100 and the second electrode plate 200.

The first electrode plate 100, the second electrode plate 200, and the elastic medium 300 form one variable capacitor that varies with the pressure. A calculation formula for the variable capacitor is:

$$C = \frac{\varepsilon_0 \times \varepsilon \times S}{d}$$

C denotes the capacitance of the variable capacitor, measured in farads (F); $\varepsilon_0$ denotes a vacuum dielectric constant; $\varepsilon$ denotes a relative dielectric constant of the elastic medium 300; S denotes the relative effective area of the first electrode plate 100 and the second electrode plate 200; d denotes the distance between the first electrode plate 100 and the second electrode plate 200.

When the flexible sensor 10 is subjected to the pressure, the elastic medium 300 is accordingly deformed to a certain extent, which causes the distance between the first electrode plate 100 and the second electrode plate 200 to change to a certain extent, thereby enabling the capacitance of the variable capacitor to change. The flexible sensor 10 forms the variable capacitor. The flexible sensor 10 is electrically connected to an external processing circuit (which is not shown in the figure). The flexible sensor 10 converts the change amount of the pressure into a change in the capacitance and outputs a signal to the external processing circuit. The external processing circuit may detect a change in the output capacitance to determine whether the pressure is applied and the magnitude of the pressure. The first electrode plate 100 is a first output terminal of the flexible sensor 10, and the second electrode plate 200 is a second output terminal of the flexible sensor 10.

Exemplarily, to prevent the first electrode plate 100 and the second electrode plate 200 on the two sides of the elastic medium 300 from being displaced when the flexible sensor 10 is subjected to the pressure, the first electrode plate 100 and the second electrode plate 200 may be secured to the two sides of the elastic medium 300 through an adhesive tape connection and/or sewing connection, which is convenient to design into various shapes to be hidden in intelligent furniture. When a user uses the intelligent furniture provided with the flexible sensor 10, the intelligent furniture may detect, through the flexible sensor 10, whether the intelligent furniture is subjected to pressure from the user, thereby determining whether the user is using the intelligent furniture.

The multiple first conductive strips 110 of the first electrode plate 100 are spaced apart from each other to avoid false detection. For example, when there is a pet at home, the pet may step on the intelligent furniture and generally has slender limbs. The multiple first conductive strips 110 spaced apart can appropriately reduce the sensitivity of the flexible sensor 10 for performing screening detection, thereby only detecting the pressure from the user and filtering out the pressure from the pet. Additionally, the multiple first conductive strips 110 spaced apart can reduce the cost and improve the bendability of the first electrode plate 100, allowing larger angles at intervals. When the flexible sensor 10 is subjected to a large pressure, the deformation of the elastic medium 300 is large, and the multiple first conductive strips 110 spaced apart can reduce some pulling force and improve the tensile resistance of the flexible sensor 10. For example, the flexible sensor 10 may be applied to intelligent furniture that is subjected to pulling, such as a mattress, a couch, and a car seat cushion, to detect whether the user is using the intelligent furniture.

In the embodiment of the present application, the variable capacitor is formed by the first electrode plate, the second electrode plate, and the elastic medium to perform the pressure detection, which has a simple structure, a low detection cost, a reliable operation, and a good dynamic performance. If a sensor applied to the intelligent furniture, such as a mattress, a couch, or a car seat cushion, has an excessively high sensitivity and is too sensitive to vibration or pressure, the false detection is prone to occur. In the present application, the multiple first conductive strips spaced apart can not only appropriately reduce the sensitivity of the flexible sensor and avoid the false detection but also improve the bendability and tensile resistance of the flexible sensor and have a low manufacturing cost. Moreover, the flexible sensor can make the user feel comfortable with no foreign body sensation when being applied to the intelligent furniture such as a mattress, a couch and a car seat cushion, thereby facilitating the wide promotion and application.

Optionally, FIG. 2 is a diagram illustrating the bottom structure of a flexible sensor according to an embodiment of the present application. As shown in FIG. 2, the second electrode plate 200 includes an integral piece of conductive cloth.

The conductive cloth uses fiber cloth (commonly polyester fiber cloth) as the base material. After a pre-treatment, the fiber cloth is electroplated with a metal coating to make the fiber cloth metallic to become the conductive fiber cloth. The conductive cloth is widely used for making high-radiation working clothes for electronics, electromagnetics and other industries. In the embodiment of the present application, radiation-proof conductive cloth is used as the electrode plate of the flexible sensor, which is foldable, washable, and low-cost.

Exemplarily, referring to FIGS. 1 and 2, using the adhesive tape connection as an example, the first electrode plate 100 is optionally the multiple first conductive strips 110 with adhesive backing, such as conductive cloth adhesive tapes, and the second electrode plate 200 is optionally radiation-proof conductive cloth with adhesive backing. The first electrode plate 100 and the second electrode plate 200 are pasted on the two opposite sides of the elastic medium 300 respectively, which is simple to manufacture and has a low production cost. The conductive cloth adhesive tapes of the multiple first conductive strips 110 and the conductive cloth of the second electrode plate 200 may be made of the same material and formed by cutting the integral piece of conductive cloth. Additionally, when there is an interference signal from the outside, the integral piece of conductive cloth can protect an output signal of the first electrode plate 100 from being easily interfered with, thereby improving the reliability and accuracy of the flexible sensor 10.

In the embodiment of the present application, the second electrode plate 200 includes the integral piece of conductive cloth so that the second electrode plate 200 can always face the multiple first conductive strips 110 of the first electrode plate 100, thereby preventing missed detection due to misalignment of the first electrode plate 100 and the second electrode plate 200 when the elastic medium 300 is deformed and improving the accuracy of a detection result. Additionally, the second electrode plate 200 can isolate the interference signal, prevent the detection result from being affected due to interference with the output signal of the first electrode plate 100, and improve the accuracy of the detection result.

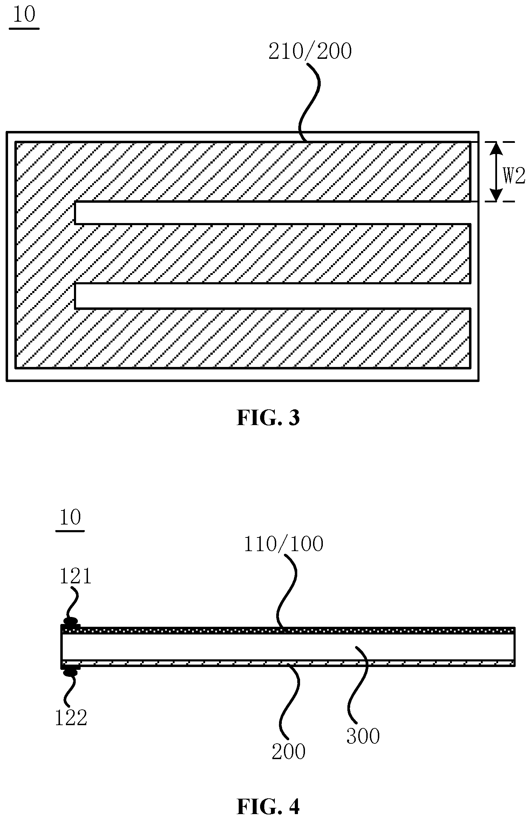

Optionally, FIG. 3 is another diagram illustrating the bottom structure of a flexible sensor according to an embodiment of the present application. Referring to FIGS. 1 and 3, the second electrode plate 200 includes multiple second conductive strips 210 in one-to-one correspondence with the multiple first conductive strips 110. The width W2 of a second conductive strip 210 is greater than the width W1 of a first conductive strip 110.

Exemplarily, when the flexible sensor 10 is subjected to the pressure, the elastic medium 300 is deformed, and the first electrode plate 100 and the second electrode plate 200 are prone to misalignment in a region subjected to the pressure. The width W2 of the second conductive strip 210 is greater than the width W1 of the first conductive strip 110 so that the multiple first conductive strips 110 can still be in one-to-one correspondence with the multiple second conductive strips 210 after misalignment, and no missed detection can be caused by the misalignment. Moreover, the second electrode plate 200 includes the multiple second conductive strips 210 that are spaced apart and in one-to-one correspondence with the multiple first conductive strips 110, which can reduce the sensitivity of the flexible sensor 10 and reduce the false detection rate. Additionally, the cost can also be reduced by reducing the use of materials.

Optionally, with continued reference to FIGS. 1 and 3, the difference between the width W2 of the second conductive strip 210 and the width W1 of the first conductive strip 110 is positively correlated with the thickness d of the elastic medium 300.

Exemplarily, when the flexible sensor 10 is subjected to the pressure, the larger the thickness d of the elastic medium 300, the larger the degree of the misalignment of the first electrode plate 100 and the second electrode plate 200 in the region subjected to the pressure. If the difference between the width W2 of the second conductive strip 210 and the width W1 of the first conductive strip 110 increases with the thickness d of the elastic medium 300 in this case, the multiple first conductive strips 110 and the multiple second conductive strips 210 that are greatly misaligned can still be in one-to-one correspondence, thereby avoiding the missed detection and improving the accuracy of the detection result.

Optionally, referring to FIGS. 1 to 3, the first electrode plate 100 is located on a side of the elastic medium 300 facing the user, and the second electrode plate 200 is located on the other side of the elastic medium 300 facing away from the user.

Exemplarily, if the second electrode plate 200 is located on the side of the elastic medium 300 facing the user, and the second electrode plate 200 includes the integral piece of conductive cloth or the multiple second conductive strips 210 with widths greater than those of the multiple first conductive strips 110, the area of the second electrode plate 200 is greater than that of the first electrode plate 100; the second electrode plate 200 is subjected to a large pulling force when the flexible sensor 10 is subjected to the pressure. If the first electrode plate 100 is located on the side of the elastic medium 300 facing the user, the first electrode plate 100 is subjected to a small pulling force when the flexible sensor 10 is subjected to the pressure, which can prolong the service life of the flexible sensor 10.

Optionally, FIG. 4 is a diagram illustrating the cross-sectional structure of a flexible sensor according to an embodiment of the present application. As shown in FIG. 4, the flexible sensor 10 further includes a first electrode button 121 and a second electrode button 122. The first electrode plate 100 is electrically connected to the external processing circuit (which is not shown in the figure) through the first electrode button 121, and the second electrode plate 200 is electrically connected to the external processing circuit through the second electrode button 122. The type of the first electrode button 121 and the second electrode button 122 are not limited in the embodiment of the present application and may be male buttons or female buttons. For ease of description, both the first electrode button 121 and the second electrode button 122 being male buttons is used as an example.

Exemplarily, the flexible sensor 10 is electrically connected to the external processing circuit of the flexible sensor 10 through the first electrode button 121 and the second electrode button 122. When both the first electrode button 121 and the second electrode button 122 are the male buttons, the first electrode button 121 and the second electrode button 122 may be electrically connected to the external processing circuit through female buttons. The first electrode button 121 and the second electrode button 122 can facilitate the replacement of the flexible sensor 10 and the replacement of the external processing circuit so that the flexible sensor 10 can be applied to more usage scenarios. The first electrode button 121 may be at any position of the flexible sensor 10, and the second electrode button 122 may be at any position of the flexible sensor 10. The flexible sensor 10 may include multiple first electrode buttons 121 and multiple second electrode buttons 122. The first electrode button and the second electrode button are two output terminals of the variable capacitor, which facilitates the connection of the flexible sensor 10 to the external processing circuit. Generally, to improve user comfort and circuit simplicity, the first electrode button 121 may be secured to a position of the first electrode plate 100 near the edge of the flexible sensor 10, and the second electrode button 122 may be secured to a position of the second electrode plate 200 near the edge of the flexible sensor 10.

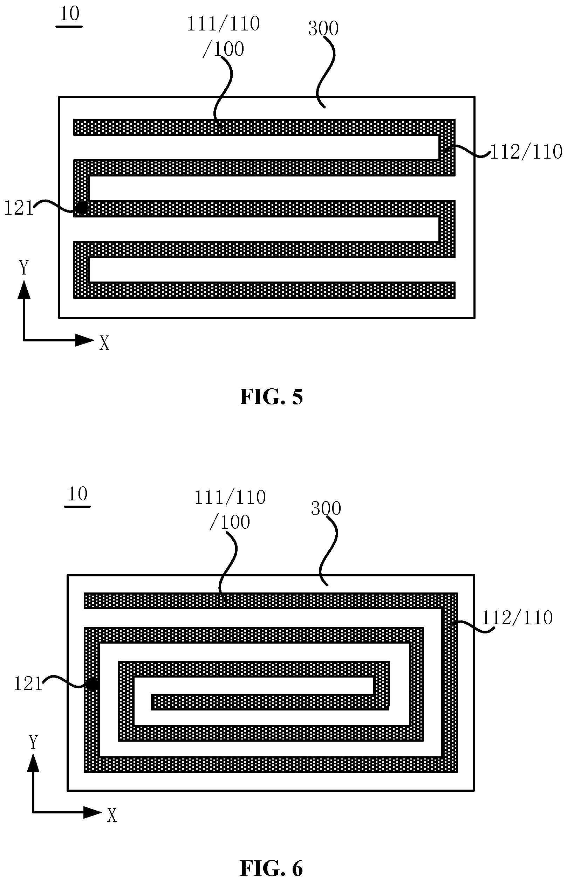

Optionally, FIG. 5 is a diagram illustrating the top structure of a flexible sensor according to an embodiment of the present application. As shown in FIG. 5, the multiple first conductive strips 110 include first conductive sub-strips 111 and second conductive sub-strips 112; the first conductive sub-strips 111 extend in the first direction X and are arranged in the second direction Y; adjacent first conductive sub-strips 111 are connected by the second conductive sub-strips 112.

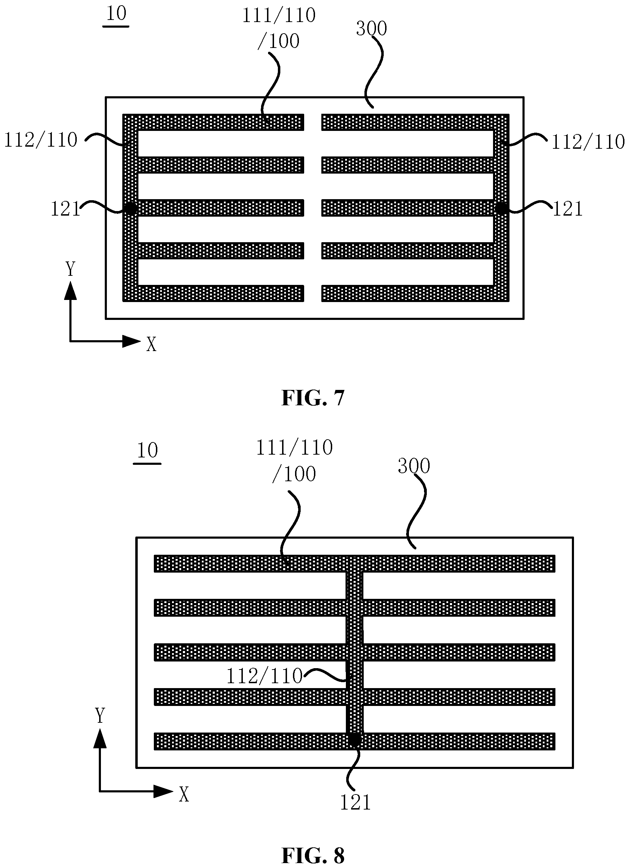

Exemplarily, referring to FIG. 5, the second conductive sub-strips 112 extend in the second direction Y, and the adjacent first conductive sub-strips 111 are connected end to end through the second conductive sub-strips 112 so that the multiple first conductive strips 110 can be distributed in a serpentine shape; the first electrode button 121 may be secured to a first conductive strip 110 close to or located in the middle among the multiple first conductive strips 110 so that a current can be distributed in the first electrode plate 100 uniformly; the first electrode button 121 is located at a position of the edge of the flexible sensor 10, thereby improving the user comfort. FIG. 6 is another diagram illustrating the top structure of a flexible sensor according to an embodiment of the present application. Referring to FIG. 6, the second conductive sub-strips 112 extend in the second direction Y, and the first conductive sub-strips 111 and the second conductive sub-strips 112 are alternately connected sequentially so that the multiple first conductive strips 110 can be in a loop shape and cover the side of the elastic medium 300 sequentially in an outside-in manner. Connection manners of the first conductive sub-strips 111 and the second conductive sub-strips 112 may also be arranged as shown in FIGS. 7 and 8. In the embodiment of the present application, the second conductive sub-strips 112 are not limited, and the connection arrangement of the first conductive sub-strips 111 and the second conductive sub-strips 112 is also not limited.

Optionally, the flexible sensor 10 includes multiple first electrode plates 100. Using the flexible sensor 10 including two first electrode plates 100 in FIG. 7 as an example, the flexible sensor 10 further includes two first electrode buttons 121 that are secured to the two first electrode plates 100 respectively.

Exemplarily, the flexible sensor 10 further includes two second electrode plates (which are not shown in the figure) and may form two variable capacitors. According to the capacitance information of the two capacitors, when the flexible sensor 10 is subjected to the pressure, the pressure position information may be determined to perform a positioning function.

Optionally, the width of the first conductive strip is 1 cm ± 0.5 cm, and the spacing distance between the adjacent first conductive strips is 5 cm ± 2.5 cm.

Exemplarily, referring to FIG. 5, the width of a first conductive sub-strip 111 and the width of a second conductive sub-strip 112 are each 1 cm ± 0.5 cm; the spacing distance between adjacent first conductive sub-strips 111 is 5 cm ± 2.5 cm. The width of the first conductive strip 110 and the spacing distance between the adjacent first conductive strips 110 are reasonably designed so that the sensitivity of the flexible sensor can be adjusted. For example, the width of the first conductive strip 110 may be reduced, and/or the spacing distance between the adjacent first conductive strips 110 may be increased so that the area directly facing the first electrode plate 100 and the second electrode plate 200 can be reduced, and the sensitivity of the flexible sensor 10 can be appropriately reduced, thereby preventing the false detection; the width of the first conductive strip 110 may also be increased, and/or the spacing distance between the adjacent first conductive strips 110 may also be reduced so that the area directly facing the first electrode plate 100 and the second electrode plate 200 can be increased, and the sensitivity of the flexible sensor 10 can be appropriately improved.

Optionally, the elastic medium 300 includes a sponge insulator. With very good elasticity and support, the sponge insulator is very suitable for the intelligent furniture and can not only increase the comfort of the intelligent furniture but also disperse the pressure when the flexible sensor 10 is subjected to the pressure, thereby prolonging the service life of the flexible sensor 10. Additionally, the sponge insulator has very good rebound resilience. When the pressure on the flexible sensor 10 disappears, the distance between the first electrode plate 100 and the second electrode plate 200 may be restored to the distance state when the flexible sensor 10 is subjected to no pressure by use of the rebound resilience of the sponge insulator, and the capacitance of the variable capacitor is also restored to the state when the flexible sensor 10 is subjected to no pressure. Moreover, the sponge insulator has a low cost, which reduces the cost of the sensor.

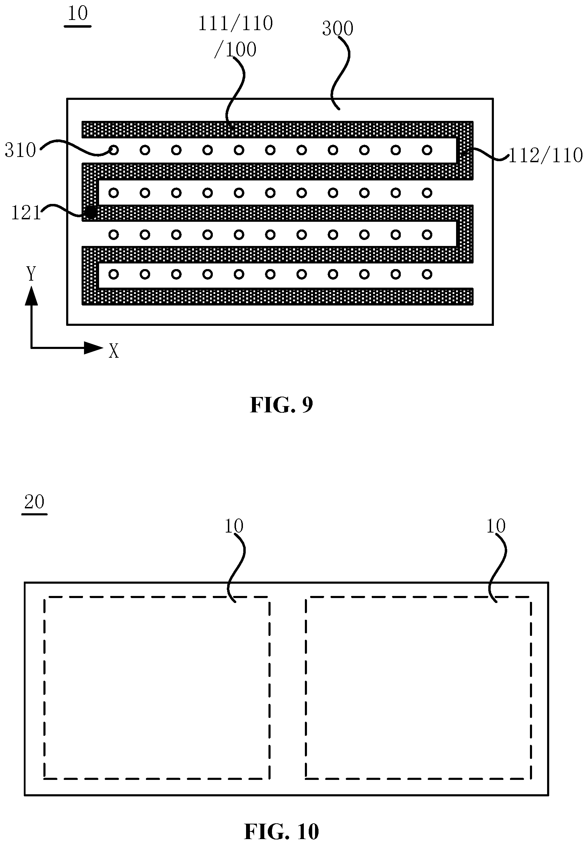

Optionally, FIG. 9 is another diagram illustrating the top structure of a flexible sensor according to an embodiment of the present application. As shown in FIG. 9, the elastic medium 300 is provided with multiple air vents 310, and the multiple air vents 310 and the multiple first conductive strips 110 do not overlap each other in the thickness direction of the flexible sensor 10. The multiple air vents 310 facilitate air ventilation and improve the user comfort.

An embodiment of the present application further provides an intelligent mat. FIG. 10 is a diagram illustrating the structure of an intelligent mat according to an embodiment of the present application. As shown in FIG. 10, the intelligent mat 20 includes the flexible sensor 10 provided in the embodiment of the present application. For example, the intelligent mat may be a mattress or a seat cushion and applied to scenarios such as an intelligent bed and a car seat. For example, when the intelligent mat is applied to the intelligent bed, the user's time in and out of bed may be monitored; when the intelligent mat is applied to the car seat cushion, the number of seats currently occupied by people may be automatically identified.

Optionally, with continued reference to FIG. 10, the intelligent mat 20 may include multiple flexible sensors 10; each flexible sensor 10 is electrically connected to the external processing circuit. The external processing circuit is configured to determine the pressure position information of the intelligent mat according to the capacitance information of each flexible sensor 10.

Exemplarily, the intelligent mat 20 may be applied to the intelligent couch. The length of the intelligent couch and the width of the intelligent couch are quite different. One intelligent mat 20 may be provided with the multiple flexible sensors 10. Which positions of the intelligent couch are occupied is determined through the capacitance information of each flexible sensor 10, and then those positions are automatically heated when the room temperature is low.

The intelligent mat provided in the embodiment of the present application may include the flexible sensor provided in any one of the embodiments of the present application and has corresponding structures and effects.

## Claims

1. A flexible sensor, configured to be electrically connected to an external processing circuit and comprising: a first electrode plate, a second electrode plate, and an elastic medium,

   wherein the first electrode plate comprises a plurality of first conductive strips spaced apart; adjacent first conductive strips of the plurality of first conductive strips are electrically connected to each other;
   wherein the first electrode plate and the second electrode plate are disposed on two opposite sides of the elastic medium; the second electrode plate and the plurality of first conductive strips form a variable capacitor with the elastic medium as an insulating layer; and

wherein in a case where the elastic medium is subjected to pressure, the elastic medium is configured to undergo elastic deformation to change a distance between the first electrode plate and the second electrode plate.

2. The flexible sensor of claim 1, wherein the second electrode plate comprises an integral piece of conductive cloth.

3. The flexible sensor of claim 1, wherein the second electrode plate comprises a plurality of second conductive strips in one-to-one correspondence with the plurality of first conductive strips, wherein a width of a second conductive strip of the plurality of second conductive strips is greater than a width of a first conductive strip of the plurality of first conductive strips.

4. The flexible sensor of claim 3, wherein a difference between the width of the second conductive strip and the width of the first conductive strip is positively correlated with a thickness of the elastic medium.

5. The flexible sensor of claim 1, wherein the first electrode plate is located on a side of the elastic medium facing a user; the second electrode plate is located on a side of the elastic medium facing away from the user.

6. The flexible sensor of claim 1, further comprising: a first electrode button and a second electrode button, wherein the first electrode plate is configured to be electrically connected to the external processing circuit through the first electrode button; the second electrode plate is configured to be electrically connected to the external processing circuit through the second electrode button.

7. The flexible sensor of claim 1, wherein the plurality of first conductive strips comprise first conductive sub-strips and second conductive sub-strips; and

the first conductive sub-strips extend in a first direction and are arranged in a second direction; adjacent ones of the first conductive sub-strips are electrically connected through the second conductive sub-strips.

8. The flexible sensor of claim 1, wherein the plurality of first conductive strips each have a width of 1 cm $\pm$ 0.5 cm; a spacing distance between the adjacent first conductive strips is 5 cm $\pm$ 2.5 cm.

9. The flexible sensor of claim 1, wherein the elastic medium comprises a sponge insulator.

10. The flexible sensor of claim 1, wherein the elastic medium is provided with a plurality of air vents; the plurality of air vents and the plurality of first conductive strips do not overlap each other in a thickness direction of the flexible sensor.

11. An intelligent mat, comprising at least one flexible sensor according to any one of claims 1 to 10.

12. The intelligent mat of claim 11, comprising a plurality of flexible sensors,
wherein each flexible sensor of the plurality of flexible sensors is configured to be electrically connected to an external processing circuit; the external processing circuit is configured to determine pressure position information of the intelligent mat according to capacitance information of each flexible sensor.

<u>10</u>

110/100

W1

d

300

200

**FIG. 1**

<u>10</u>

200

**FIG. 2**

10

210/200

W2

**FIG. 3**

10

110/100

121

122

200

300

**FIG. 4**

**FIG. 5**

**FIG. 6**

**FIG. 7**

**FIG. 8**

FIG. 9

FIG. 10

## INTERNATIONAL SEARCH REPORT

| International application No. |
| --- |
| **PCT/CN2022/101135** |

| A. | CLASSIFICATION OF SUBJECT MATTER |
| --- | --- |

G01L 1/14(2006.01)i;  G01B 7/00(2006.01)i;  A47C 17/86(2006.01)i;  A47C 23/00(2006.01)i;  A47C 27/14(2006.01)i

According to International Patent Classification (IPC) or to both national classification and IPC

| B. | FIELDS SEARCHED |
| --- | --- |

Minimum documentation searched (classification system followed by classification symbols)

G01L; G01B; A47C

Documentation searched other than minimum documentation to the extent that such documents are included in the fields searched

Electronic data base consulted during the international search (name of data base and, where practicable, search terms used)

CNKI, CNPAT, EPODOC, WPI: 慕思健康, 王炳坤, 电容, 柔性 OR 弹性, 传感, 叉指 OR 回形 OR 螺旋 OR 螺线 OR 导电带, flexible, elastic+, sensor?, electrod+, press+

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 111735561 A (HEBEI UNIVERSITY OF TECHNOLOGY) 02 October 2020 (2020-10-02)<br>  description, paragraphs [0017]-[0026], and figures 1, 4, and 6 | 1-12 |
| X | CN 113970392 A (ANHUI UNIVERSITY) 25 January 2022 (2022-01-25)<br>  description, paragraphs [0027]-[0033], and figures 1-4 | 1-12 |
| X | CN 206249279 U (SHANGHAI TIANMA MICROELECTRONICS CO., LTD. et al.) 13 June 2017 (2017-06-13)<br>  description, paragraphs [0047]-[0053], and figures 1-2 | 1-12 |
| X | US 2017160854 A1 (LUMINOUS OPTICAL TECHNOLOGY CO., LTD.) 08 June 2017 (2017-06-08)<br>  description, paragraphs [0022] and [0030]-[0034], and figures 1-4 | 1-12 |
| A | CN 214149645 U (SHENZHEN INSTITUTES OF ADVANCED TECHNOLOGY, CHINESE ACADEMY OF SCIENCES) 07 September 2021 (2021-09-07)<br>  entire document | 1-12 |
| A | US 2005215915 A1 (SANYO ELECTRIC CO., LTD.) 29 September 2005 (2005-09-29)<br>  entire document | 1-12 |

☐ Further documents are listed in the continuation of Box C.          ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **18 November 2022** | **29 November 2022** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

<table>
<tr><td colspan="2" align="center">INTERNATIONAL SEARCH REPORT<br>Information on patent family members</td><td colspan="2">International application No.<br><br>**PCT/CN2022/101135**</td></tr>
<tr><td>Patent document<br>cited in search report</td><td>Publication date<br>(day/month/year)</td><td>Patent family member(s)</td><td>Publication date<br>(day/month/year)</td></tr>
<tr><td>CN  111735561  A</td><td>02 October 2020</td><td>None</td><td></td></tr>
<tr><td>CN  113970392  A</td><td>25 January 2022</td><td>None</td><td></td></tr>
<tr><td>CN  206249279  U</td><td>13 June 2017</td><td>None</td><td></td></tr>
<tr><td>US  2017160854  A1</td><td>08 June 2017</td><td>None</td><td></td></tr>
<tr><td>CN  214149645  U</td><td>07 September 2021</td><td>None</td><td></td></tr>
<tr><td>US  2005215915  A1</td><td>29 September 2005</td><td>JP  2005315831  A<br>US  7641618  B2<br>JP  4141426  B2<br>JP  2008241717  A</td><td>10 November 2005<br>05 January 2010<br>27 August 2008<br>09 October 2008</td></tr>
</table>

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210418377